# EUROPEAN PATENT APPLICATION

(11) **EP 4 811 272 A1**
(43) Date of publication of application: **23.09.2026**
(21) Application number: 26165857.9
(22) Date of filing: 18.03.2026
(51) Int. Cl.: G06T 5/60, A61B 6/00, G06N 3/02

(54) **MEDICAL IMAGE PROCESSING METHOD AND SYSTEM, DEVICE AND MEDIUM**

(30) Priority: 19.03.2025 CN 202510331744
(71) Applicant: Siemens Healthineers AG, 91301 Forchheim (DE)
(72) Inventor: LU, Yi Dan, Shanghai, SH 201318 (CN); HU, Fei, Shanghai, SH 201318 (CN)
(74) Representative: HKW Intellectual Property PartG mbB

(57) **Abstract**

The present application provides a medical image processing method and system, a device and a medium, for acquiring raw data; subjecting the raw data to image reconstruction, to generate an image to be processed; preprocessing the image to be processed; inputting the preprocessed image to be processed into a trained image optimization model to obtain a target image, such that a resolution of the target image is higher than a resolution of the image to be processed; wherein the image optimization model is obtained by training a diffusion model, a training process of the diffusion model having high-dose high-resolution image data as a starting point; the raw data originates in a first device, the high-dose high-resolution image data used to train the diffusion model originates in a second device, and the second device has better image processing capability than the first device. The present application can significantly improve medical image quality at a low cost.

## Description

### TECHNICAL FIELD

The present application relates to the technical field of medical image processing, in particular to a medical image processing method and system, a device and a medium.

### BACKGROUND ART

Computed Tomography (CT) is an imaging method widely used for clinical diagnosis. An example is CT angiography of the coronary arteries (Coronary Computed Tomography Angiography, CCTA), which uses CT scan techniques to generate detailed images of heart blood vessels, helping doctors to identify atherosclerosis, stenosis and other problems that might lead to heart attacks. However, although CCTA is of considerable value, certain challenges are still faced in its application. First of all, blooming artifacts are a key issue affecting CCTA image quality. This phenomenon typically appears around high-density matter, e.g. calcified plaques in the coronary arteries, causing these regions to appear larger or fuzzier than they really are, and thus possibly misleading diagnosis results. In addition, limited spatial resolution is also an important factor limiting CCTA accuracy. Low spatial resolution means that small lesions or early changes might not be able to be captured clearly, affecting the early discovery of illnesses and the formulation of treatment plans.

Although technological progress in recent years, such as photon-counting CT, has been able to provide images with ultra-high resolution, significantly improving image quality and reducing artifacts, this technology is currently struggling to achieve widespread application globally due to its high cost. Most medical institutions still rely on conventional CT equipment to perform clinical examinations. Thus, the development of a technology capable of significantly improving the quality of conventional CT images appears to be especially important. Progress in this technology is expected to use lower medical costs, while helping to significantly improve the precision and reliability of diagnosis.

### SUMMARY OF THE INVENTION

In view of the shortcomings in the prior art mentioned above, an objective of the present application is to provide a computed tomography image processing method and system, a device and a medium, to solve one or more technical problems in the prior art.

To achieve the above objective and other related objectives, the present application provides a medical image processing method, comprising:
acquiring raw data;
subjecting the raw data to image reconstruction, to generate an image to be processed;
preprocessing the image to be processed;
inputting the preprocessed image to be processed into a trained image optimization model to obtain a target image, such that a resolution of the target image is higher than a resolution of the image to be processed;
wherein the image optimization model is obtained by training a diffusion model, a training process of the diffusion model having high-dose high-resolution image data as a starting point and target high-resolution image data as an endpoint of a diffusion process; the raw data originates in a first device, the high-dose high-resolution image data used to train the diffusion model originates in a second device, the second device has better image processing capability than the first device, and under equivalent external conditions, an image generated by the second device has higher resolution than an image generated by the first device.

In some embodiments of the present application, the second device is photon-counting CT, and/or the first device is non-photon-counting CT.

In some embodiments of the present application, the equivalent external conditions comprise identical radiation doses used during imaging.

In some embodiments of the present application, preprocessing the image to be processed comprises:
extracting image pixel data from the image to be processed;
using an interpolation method to process the pixel data into virtual high-resolution image data;
subjecting the virtual high-resolution image data to normalization.

In some embodiments of the present application, the image optimization model is obtained by training a diffusion model, and training of the diffusion model comprises:
constructing a multi-dose training dataset, comprising multiple items of respectively paired high-dose high-resolution data and low-dose normal-resolution data, the high-dose high-resolution data and the low-dose normal-resolution data both originating in the second device.

In some embodiments of the present application, the low-dose normal-resolution data is processed into low-dose simulated high-resolution image data by an interpolation method, and paired with the high-dose high-resolution data to serve as training input data and a target image.

In some embodiments of the present application, the training process of the diffusion model comprises:
using a mean-preserving degradation operator to gradually add noise to the high-dose high-resolution image data via a diffusion process, so that it degrades to corresponding low-dose low-resolution image data, then gradually eliminating noise from the low-dose low-resolution image data via a reverse diffusion process to generate high-resolution image data; and through continual training, gradually bringing the high-resolution image data closer to the high-dose high-resolution image data to serve as target high-resolution image data.

The present application further provides a medical image processing system, comprising:
a medical image acquisition apparatus, used for raw data and defined as a first device;
a processor, configured to:
   subject the raw data to image reconstruction, to generate an image to be processed;
   preprocess the image to be processed;
   input the preprocessed image to be processed into a trained image optimization model to obtain a target image, such that a resolution of the target image is higher than a resolution of the image to be processed;
   wherein the image optimization model is obtained by training a diffusion model, a training process of the diffusion model having high-dose high-resolution image data as a starting point and target high-resolution image data as an endpoint of a diffusion process; the raw data originates in a first device, the high-dose high-resolution image data used to train the diffusion model originates in a second device, the second device has better image processing capability than the first device, and under equivalent external conditions, an image generated by the second device has higher resolution than an image generated by the first device.

The present application further provides an electronic device, comprising:
one or more processor;
a storage means for storing one or more program which, when executed by the one or more processor, causes the electronic device to realize the medical image processing method as described above.

The present application further provides a computer-readable storage medium, having stored thereon a computer program which, when executed by a processor of a computer, causes the computer to perform the medical image processing method as described above.

The medical image processing method and system, device and medium provided in the present application have at least the following beneficial effects:
Firstly, the focus of conventional training models is changed from continual denoising to adjustment of the input data source. That is, all data of the training model is generated by a second device with higher image processing capability, for example by photon-counting CT which has newly entered the market at the present time. This ensures the quality of high-resolution DICOM images and the entire training dataset.

Secondly, the present application introduces a diffusion model as a deblurring and noise reduction network, which can effectively distinguish between noise and meaningful structural information in an image, so as to retain important edges and details as much as possible while removing noise, and can reduce or eliminate artifacts caused by the imaging process more effectively, e.g. phenomena such as fuzziness caused by the partial volume effect, thus increasing the overall clarity and readability of the image.

Furthermore, although imaging devices which have newly appeared, such as photon-counting CT, have excellent imaging capability and can obtain high-quality images with ultra-high resolution, they currently have a high application cost and are thus struggling to rapidly cover market demand. In the diagnosis of certain specific symptoms especially, the particular demand for high-quality images with ultra-high resolution is very pressing; an example is coronary CT angiography. The inventors of the present application have creatively solved this technical problem, using a second device with better imaging capability, such as that mentioned above, as a training data source, so that low-resolution medical image data provided by an ordinary first device such as that mentioned above can be processed into a high-quality image having high resolution. This high-resolution high-quality image is equivalent to one generated by the second device, so more accurate diagnosis is provided for the patient at a low cost.

### BRIEF DESCRIPTION OF THE DRAWINGS

Fig. 1 is a schematic flow chart of a medical image processing method provided in embodiments of the present application.
Fig. 2 is a schematic flow chart of a method provided in embodiments of the present application for using a DICOM preprocessing module to preprocess the image to be processed.
Fig. 3 is a schematic diagram of a training process of a diffusion model provided in embodiments of the present application.
Fig. 4 is a schematic diagram of diffusion and reverse diffusion processes of a diffusion model provided in embodiments of the present application.
Fig. 5 is a structural schematic diagram of a medical image processing system provided in embodiments of the present application.
Fig. 6 is a structural schematic diagram of an electronic device provided in embodiments of the present application.

### DETAILED DESCRIPTION OF EMBODIMENTS

Embodiments of the present application are explained below through specific examples; those skilled in the art will be able to readily understand other advantages and effects of the present application from the content disclosed herein. The present application could also be implemented or applied by way of other, different particular embodiments, and various modifications or changes could be made to various details herein based on different viewpoints and applications without departing from the spirit of the present application. It must be explained that in the absence of conflict, the embodiments below and features therein can be combined with each other.

It must be explained that the drawings provided in the embodiments below merely illustrate the basic concept of the present application schematically. Thus, the drawings only show components relevant to the present application, and are not drawn according to the numbers, shapes and dimensions of components in actual implementation. The forms, numbers and proportions of the various components when actually implemented may be arbitrarily changed, and the layout of the components may be more complicated.

In the description below, many details are discussed to provide a more thorough explanation of embodiments of the present application. However, to those skilled in the art, it is obvious that embodiments of the present application could be implemented without these specific details. In other embodiments, well known structures and devices are shown in the form of block diagrams rather than in the form of details, to avoid making embodiments of the present application difficult to understand.

As artificial intelligence technology, especially deep learning, develops, optimization methods based on this technology are gradually being applied in the field of medical image processing, e.g. image noise reduction and spatial resolution improvement. Deep learning models can automatically learn features from large amounts of data, and are used to improve image quality. For example, spatial resolution of images is improved by learning mappings between low-resolution and high-resolution images, and noise in images is identified and removed by model training. However, much research has shown that the ability of these models to improve spatial resolution is relatively limited, and they cannot improve image resolution based on expected values. The inventors of the present application inventively discovered in the course of much experimentation that the limitations of spatial resolution improvement through model training are mainly focussed on two issues. Firstly, training data limitations: if a model is trained using conventional CT images with normal resolution, the model will not have enough information to learn how to effectively improve the spatial resolution of images. Secondly, model design tendencies: due to the limitations of image resolution improvement, many model architectures or training strategies are more inclined to optimize noise reduction tasks rather than resolution improvement. Obviously, although methods based on deep learning have shown huge potential for medical image processing, they still face challenges regarding how to effectively and significantly improve spatial resolution in specific applications.

To solve the abovementioned technical problems in the prior art, the present application inventively uses high-resolution, high-quality images as a training standard, effectively increasing the image resolution optimization height. In addition, the present application also introduces a diffusion model, using the diffusion model as an image quality improvement network, and uses a mean-preserving degradation operator to optimally reconstruct a low-resolution image. Particular solutions of the present application are presented in further detail below.

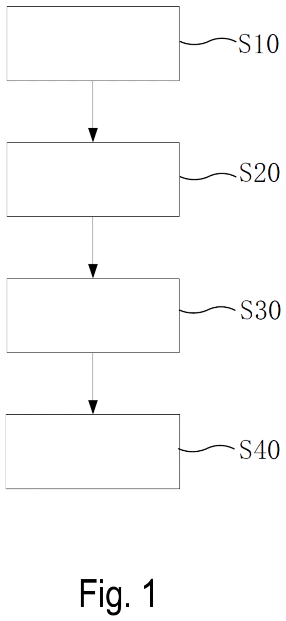

As shown in Fig. 1, the present application provides a medical image processing method, comprising the following steps:
S10, acquiring raw data;
S20, subjecting the raw data to image reconstruction, to generate an image to be processed;
S30, preprocessing the image to be processed;
S40, inputting the preprocessed image to be processed into a trained image optimization model to obtain a target image, such that a resolution of the target image is higher than a resolution of the image to be processed;
wherein the image optimization model is obtained by training a diffusion model, a training process of the diffusion model having high-dose high-resolution image data as a starting point and target high-resolution image data as an endpoint of a diffusion process; the raw data originates in a first device, the high-dose high-resolution image data used to train the diffusion model originates in a second device, the second device has better image processing capability than the first device, and under equivalent external conditions, an image generated by the second device has higher resolution than an image generated by the first device.

Specifically, in step S10, original image data is acquired from a medical image device (such as an ordinary CT scanner); more specifically, the raw data originates in a first device, which is non-photon-counting CT, or another medical imaging device with lower image processing capability than the second device. Generally, the first device has a lower cost and lower technical complexity than the second device, and the first device was applied on the market earlier than the second device. For example, the first device is conventional X-ray CT, which uses an X-ray source and a detector array, and generates an image by measuring differences in the intensity of X-rays passing through different regions of the human body, and this technology relies on the acquisition and conversion of simulation signals; the second device is photon-counting CT, which uses a more advanced detector technology, being able to count individual X-ray photons directly and form an image according to the photon energy information, thus being able to provide higher contrast resolution without any loss of spatial resolution. As will be understood by those skilled in the art, the first device and second device of the present application are not limited to non-photon-counting CT and photon-counting CT as mentioned above. As technology continually develops, it is possible that other medical imaging devices with higher image processing capability than photon-counting CT will emerge. In this case, the first device could also be a medical imaging device including photon-counting CT, and the second device is another, more advanced medical imaging device.

In step S20, the raw data is subjected to image reconstruction, to generate an image to be processed. Specifically, since the raw data is data acquired by an imaging device, the data has not yet undergone any substantive post-processing; therefore, an image reconstruction process is used to convert the raw data to a two-dimensional or three-dimensional visual image, i.e. to generate an image to be processed. As will be understood by those skilled in the art, the image reconstruction process includes Filtered Back-Projection (FBP), Iterative Reconstruction (IR), etc.

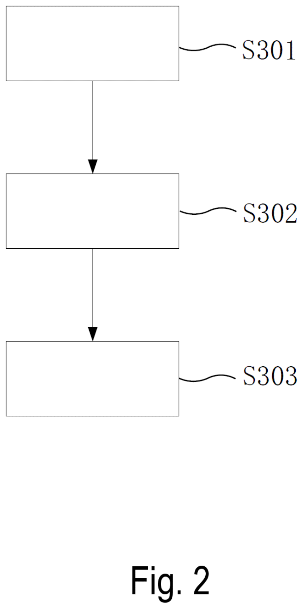

In step S30, the image to be processed is preprocessed, in order to be provided to the image optimization model. Specifically, as shown in Fig. 2, using a DICOM preprocessing module to preprocess the image to be processed comprises:
S301, extracting image pixel data from the image to be processed, e.g. extracting pixel information from a DICOM file of normal-resolution image data (such as a 512*512 array);
S302, using an interpolation method, for example a bicubic interpolation method to process the pixel data into virtual high-resolution image data (such as a 1024*1024 array);
S303, subjecting the processed data to normalization, so that all pixel values fall within a specific range (e.g. 0 to 1 or -1 to 1), in preparation for further data processing, so that a subsequent processing step can be more efficient and accurate; for example, linear normalization or Z-score standardization, etc. is used.

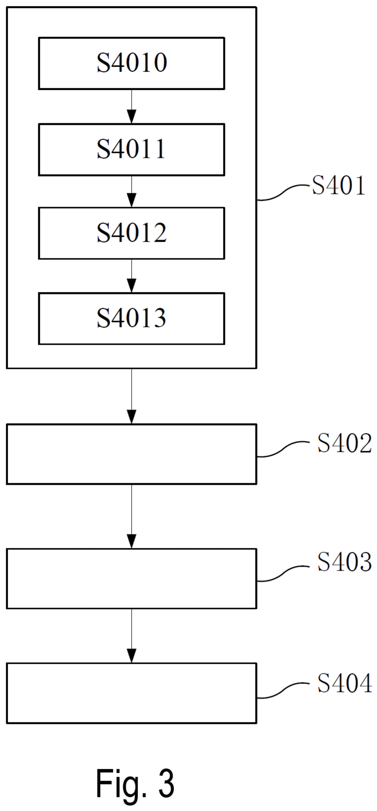

In step S40, the preprocessed image to be processed is input into a trained image optimization model to obtain a target image, such that a resolution of the target image is higher than a resolution of the image to be processed. Specifically, the image optimization model is obtained by training a diffusion model; a training process of the diffusion model has high-dose, high-resolution image data (such as a 1024*1024 array) as a starting point, and target high-resolution image data as an endpoint of a diffusion process. Referring to Fig. 3, which is a schematic drawing of the training process of the diffusion model provided in embodiments of the present application, the process comprises:
S401, constructing a multi-dose training dataset, comprising multiple items of respectively paired high-dose high-resolution data and low-dose normal-resolution data, the high-dose high-resolution data and the low-dose normal-resolution data both originating in the second device.

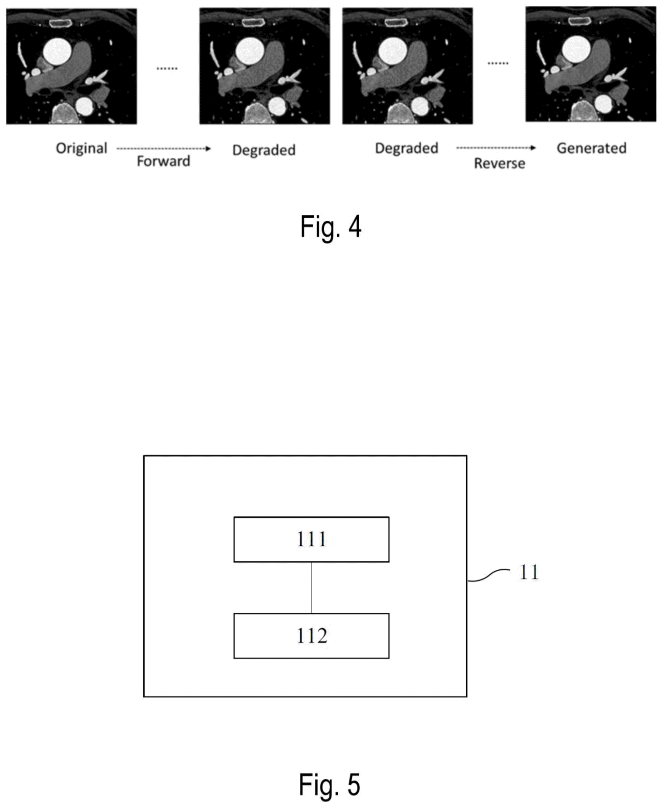

Specifically, step S401 comprises:
S4010, using a multi-dose original data collection module to collect respectively paired high-dose high-resolution data and low-dose normal-resolution data;
S4011, using a DICOM preprocessing module to extract pixel information from a DICOM file of the low-dose normal-resolution image data (such as a 512*512 array);
S4012, using an interpolation method, for example a bicubic interpolation method to process the pixel data to obtain low-dose simulated high-resolution image data (such as a 1024*1024 array);
S4013, subjecting the low-dose simulated high-resolution image data and the high-dose high-resolution data to normalization, and then pairing the normalized data to form a training dataset.
S402, inputting the training dataset into the diffusion model.
S403, training the diffusion model to obtain the image optimization model. Specifically, referring to Fig. 4, the diffusion model gradually adds noise to the high-dose high-resolution image data via a diffusion process, so that it degrades to corresponding low-dose low-resolution image data, then gradually eliminates noise from the low-dose low-resolution image data via a reverse diffusion process to generate high-resolution image data. Through continual training, the high-resolution image data is gradually brought closer to the high-dose high-resolution image data and serves as target high-resolution image data. Thus, when low-dose image data is received, the reverse process of the diffusion model can obtain a high-dose high-resolution image similar to that obtained from the second device.

More specifically, as shown in Fig. 4, the diffusion process, also called the forward process, has the high-dose high-resolution image data (the image furthest to the left in Fig. 4, where "Original" means the original image) as the starting point of diffusion, and the low-dose low-resolution image data (the middle two images in Fig. 4, where "Degraded" means a degraded image) as a point in the diffusion process. Using a mean-preserving degradation operator, noise with a mean value of zero is gradually added to the high-dose high-resolution image data so that it is continually degraded to the low-dose low-resolution image data. Thus, the number of steps of noise addition can be effectively reduced, increasing the model inference speed. Taking CT images as an example, the inference time for data of a single CT image can be reduced by 0.05 s - 0.1 s, e.g. 0.05 s, 0.075 s or 0.1 s. The mean-preserving degradation operator is defined as follows: ${x}_{t} = {α}_{t} {x}_{0} + \left(1-{α}_{t}\right) {x}_{T}$ where ***x***₀ is high-dose high-resolution CT image data; ***x**_{T}* is low-dose simulated high-resolution CT image data; T is the total number of diffusion steps; T is greater than or equal to 5 and less than or equal to 20, e.g. 5, 10, 15 or 20; t is one of 1, 2, ..., T; *αₜ* is the noise factor corresponding to step number t; *αₜ<α*_{*t*-1}, and *αₜ* is the basis. It has been found by experiment that noise added using the mean-preserving degradation operator of the present application is more consistent with the true noise distribution of a CT image in original scan data; this facilitates noise simulation and learning.

The reverse diffusion process, also called the reverse process or denoising process, trains the model with a large amount of paired image data pairs from the multi-dose training dataset, such that the diffusion model learns the reverse diffusion process from low-dose low-resolution image data (the middle two images in Fig. 4) to high-dose high-resolution data (the image furthest to the right in Fig. 4, where "Generated" means the generated image), completing the denoising training of the diffusion model, so that the trained diffusion model can use the reverse diffusion process to generate the high-dose high-resolution target image by gradual optimization from the normal-resolution image to be processed.

S404, completing the training of the diffusion model to obtain the image optimization model.

After acquiring the trained denoising diffusion model, the image optimization model is obtained, and it is then possible to input the image to be processed into the image optimization model, use the reverse diffusion process of the diffusion model to subject the image to be processed to gradual denoising optimization, obtain a high-dose high-resolution image, then subject the optimized image to image post-processing according to specific requirements, e.g. amend DICOM header file information, etc.

Based on a concept similar to that described above, as shown in Fig. 5, the present application further provides a medical image processing system 11, the medical image processing system 11 comprising a medical image acquisition apparatus 111 and a processor 112.

The medical image acquisition apparatus 111 is configured to acquire raw data, and is also defined as a first device. The medical image acquisition apparatus is for example an X-ray imaging device, a computed tomography (CT) device, a magnetic resonance imaging (MRI) device, etc.

The processor 112 is configured to:
subject the raw data to image reconstruction, to generate an image to be processed;
preprocess the image to be processed;
input the preprocessed image to be processed into a trained image optimization model to obtain a target image, such that a resolution of the target image is higher than a resolution of the image to be processed; wherein the image optimization model is obtained by training a diffusion model, a training process of the diffusion model has high-dose high-resolution image data as a starting point, and target high-resolution image data as an endpoint of a diffusion process; the raw data originates in the first device, the high-dose high-resolution image data used to train the diffusion model originates in a second device, the second device has better image processing capability than the first device, and under equivalent external conditions, an image generated by the second device has higher resolution than an image generated by the first device.

As will be understood by those skilled in the art, the processor 112 may be integrated in the first device 111, or may be arranged independently of the first device 111, and the various functions in the processor 112 may be correspondingly configured as corresponding functional modules, one or more of these functional modules being optionally integrated in the processor 112, as long as the objective of the present application can be achieved; the present application imposes no special restrictions in this respect. In addition, it should be explained that the medical image processing system 11 provided in the embodiments above and the medical image processing method provided in the embodiments above are based on the same concept; thus, the functions possessed by the processor 112 have been included in the specific embodiments of the medical image processing method and described in detail, so are not repeated here.

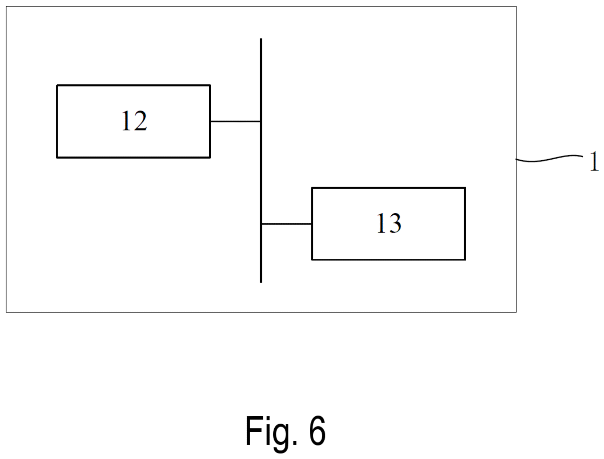

As shown in Fig. 6, the present application further provides an electronic device for realizing the medical image processing method described above.

The electronic device 1 comprises a memory 12, a processor 13 and a bus, and further comprises a computer program stored in the memory 12 and executable on the processor 13, such as a medical image processing program.

The memory 12 comprises at least one type of readable storage medium, which includes flash memory, portable hard disk, multimedia card, card memory (e.g. SD or DX memory, etc.), magnetic memory, magnetic disk, optical disk, etc. In some embodiments, the memory 12 may be an internal storage unit of the electronic device 1, e.g. a portable hard disk of the electronic device 1. In other embodiments, the memory 12 may be an external storage device of the electronic device 1, e.g. a plug-in portable hard disk provided on the electronic device 1, a smart media card (SMC), a secure digital (SD) card, a flash card, etc. Further, the memory 12 could also comprise both an internal storage unit of the electronic device 1, and an external storage device. Further, the memory could also be an interface capable of docking with a remote or virtual storage device such as cloud storage, for the purpose of transmitting information stored in these devices to the electronic device. The memory 12 may be used not only to store application software installed on the electronic device 1 and various data, such as code for medical image processing, etc., but also to temporarily store data which has already been output or will be output.

In some embodiments, the processor 13 may consist of an integrated circuit, e.g. a single packaged integrated circuit, or may consist of multiple packaged integrated circuits with identical or different functions, including one or more central processing units (CPU), microprocessors, digital processing chips, graphical processing units, and combinations of various control chips, etc. The processor 13 is a control core (control unit) of the electronic device 1, using various interfaces and circuits to connect the various components of the entire electronic device 1; it runs or executes programs or modules stored in the memory 12 (e.g. programs for computed tomography image processing, etc.), and calls data stored in the memory 12, in order to execute various functions of the electronic device 1 and process data.

The processor 13 executes an operating system of the electronic device 1 and various installed application programs. The processor 13 executes the application programs to realize the steps in the medical image processing method described above.

For example, the computer program may be divided into one or more modules; the one or more modules are stored in the memory 12, and executed by the processor 13 to realize the present application. The one or more modules may be a series of computer program instruction segments capable of performing specific functions, the instruction segments being used to describe the process of executing the computer program in the electronic device 1. For example, the computer program may be divided into functional modules respectively used for performing the steps in the medical image processing method of the present application; as those skilled in the art will understand, the present application imposes no special restrictions regarding the specific division. In addition, as those skilled in the art will understand, the processor 13 of the electronic device 1 may be the same as or different from the processor 112 of the medical image processing system, as long as the invention objective of the present application can be achieved; the present application imposes no special restrictions in this respect.

In addition, an embodiment of the present application further provides a computer-readable storage medium, having stored thereon a computer program which, when executed by a processor of a computer, causes the computer to perform the medical image processing method as described above.

In summary, the present application discloses a medical image processing method and system, a device and a medium. Raw data is acquired; the raw data is subjected to image reconstruction, to generate an image to be processed; the image to be processed is preprocessed; the preprocessed image to be processed is input into a trained image optimization model to obtain a target image, such that a resolution of the target image is higher than a resolution of the image to be processed; wherein the image optimization model is obtained by training a diffusion model, a training process of the diffusion model has high-dose high-resolution image data as a starting point, and target high-resolution image data as an endpoint of a diffusion process; in particular, the low-dose raw data originates in a first device, the high-dose high-resolution image data used to train the diffusion model originates in a second device, the second device has better image processing capability than the first device, and under equivalent external conditions, an image generated by the second device has higher resolution than an image generated by the first device. Based on the creative, breakthrough thinking of the inventors of the present application, the present application innovatively changes a conventional means of increasing medical image resolution. Firstly, the focus of conventional training models is changed from continual denoising to adjustment of the input data source. That is, all data of the training model is generated by a second device with higher image processing capability, for example by photon-counting CT which has newly entered the market at the present time. This ensures the quality of high-resolution DICOM images and the entire training dataset. Secondly, the present application introduces a diffusion model as a deblurring and noise reduction network, which can effectively distinguish between noise and meaningful structural information in an image, so as to retain important edges and details as much as possible while removing noise, and can reduce or eliminate artifacts caused by the imaging process more effectively, e.g. phenomena such as fuzziness caused by the partial volume effect, thus increasing the overall clarity and readability of the image. Furthermore, although imaging devices which have newly appeared, such as photon-counting CT, have excellent imaging capability and can obtain high-quality images with ultra-high resolution, they currently have a high application cost and are thus struggling to rapidly cover market demand. In the diagnosis of certain specific symptoms especially, the particular demand for high-quality images with ultra-high resolution is very pressing; an example is coronary CT angiography. The inventors of the present application have creatively solved this technical problem, using a second device with better imaging capability, such as that mentioned above, as a training data source, so that an image to be processed, having standard resolution and provided by an ordinary first device such as that mentioned above, can be processed into a high-quality image having high resolution. This high-resolution high-quality image is equivalent to one generated by the second device, so more accurate diagnosis is provided for the patient at a low cost, i.e. a model is trained using data generated by the second device which has a higher cost, and the model is applied to the low-cost first device, thus significantly improving the image quality of the low-cost device.

The above embodiments are merely illustrative of the principles and effects of the present application and are not intended to limit the present application. Anyone familiar with the technology may modify or change the above embodiments without violating the spirit and scope of the present application. Therefore, all equivalent modifications or changes made by those of ordinary knowledge in the relevant technical field without departing from the spirit and technical ideas disclosed in the present application shall still be covered by the claims of the present application.

## Claims

1. A medical image processing method, **characterized by** comprising:
acquiring raw data;
subjecting the raw data to image reconstruction, to generate an image to be processed;
preprocessing the image to be processed;
inputting the preprocessed image to be processed into a trained image optimization model to obtain a target image, such that a resolution of the target image is higher than a resolution of the image to be processed;
wherein the image optimization model is obtained by training a diffusion model, a training process of the diffusion model having high-dose high-resolution image data as a starting point and target high-resolution image data as an endpoint of a diffusion process; the raw data originates in a first device, the high-dose high-resolution image data used to train the diffusion model originates in a second device, the second device has better image processing capability than the first device, and under equivalent external conditions, an image generated by the second device has higher resolution than an image generated by the first device.

2. The medical image processing method as claimed in claim 1, **characterized in that** the second device is photon-counting CT, and/or the first device is non-photon-counting CT.

3. The medical image processing method as claimed in claim 1, **characterized in that** the equivalent external conditions comprise identical radiation doses used during imaging.

4. The medical image processing method as claimed in claim 1, **characterized in that** preprocessing the image to be processed comprises:
extracting image pixel data from the image to be processed;
using an interpolation method to process the pixel data into virtual high-resolution image data;
subjecting the virtual high-resolution image data to normalization.

5. The medical image processing method as claimed in claim 1, **characterized in that** the image optimization model is obtained by training a diffusion model, and training of the diffusion model comprises:
constructing a multi-dose training dataset, comprising multiple items of respectively paired high-dose high-resolution data and low-dose normal-resolution data, the high-dose high-resolution data and the low-dose normal-resolution data both originating in the second device.

6. The medical image processing method as claimed in claim 5, **characterized in that** the low-dose normal-resolution data is processed into low-dose simulated high-resolution image data by an interpolation method, and paired with the high-dose high-resolution data to serve as training input data and a target image.

7. The medical image processing method as claimed in claim 5, **characterized in that** the training process of the diffusion model comprises:
using a mean-preserving degradation operator to gradually add noise to the high-dose high-resolution image data via a diffusion process, so that it degrades to corresponding low-dose low-resolution image data, then gradually eliminating noise from the low-dose low-resolution image data via a reverse diffusion process to generate high-resolution image data; and through continual training, gradually bringing the high-resolution image data closer to the high-dose high-resolution image data to serve as target high-resolution image data.

8. A medical image processing system, **characterized by** comprising:
a medical image acquisition apparatus, configured to acquire raw data and defined as a first device;
a processor, configured to:
subject the raw data to image reconstruction, to generate an image to be processed;
preprocess the image to be processed;
input the preprocessed image to be processed into a trained image optimization model to obtain a target image, such that a resolution of the target image is higher than a resolution of the image to be processed;
wherein the image optimization model is obtained by training a diffusion model, a training process of the diffusion model having high-dose high-resolution image data as a starting point and target high-resolution image data as an endpoint of a diffusion process; the raw data originates in a first device, the high-dose high-resolution image data used to train the diffusion model originates in a second device, the second device has better image processing capability than the first device, and under equivalent external conditions, an image generated by the second device has higher resolution than an image generated by the first device.

9. An electronic device, **characterized by** comprising:
one or more processor;
a storage means for storing one or more program which, when executed by the one or more processor, causes the electronic device to realize the method as claimed in any one of claims 1 - 7.

10. A computer-readable storage medium, **characterized by** having stored thereon a computer program which, when executed by a processor of a computer, causes the computer to perform the method as claimed in any one of claims 1 - 7.
